# EUROPEAN PATENT SPECIFICATION

(11) **EP 3 995 118 B1**
(45) Date of publication and mention of the grant of the patent: **19.02.2025**
(21) Application number: 20835570.1
(22) Date of filing: 08.05.2020
(51) Int. Cl.: A61F 13/15, A61F 13/53, A61F 13/532

(54) **METHOD OF PRODUCING ABSORBENT BODY FOR ABSORBENT ARTICLE, AND ABSORBENT ARTICLE**
VERFAHREN ZUR HERSTELLUNG EINES SAUGFÄHIGEN KÖRPERS FÜR EINEN SAUGFÄHIGEN ARTIKEL UND SAUGFÄHIGER ARTIKEL
PROCEDE DE PRODUCTION DE CORPS ABSORBANT POUR ARTICLE ABSORBANT, ET ARTICLE ABSORBANT

(30) Priority: 03.07.2019 JP 2019124825
(43) Date of publication of application: 11.05.2022
(73) Proprietor: Zuiko Corporation, Osaka 566-0045 (JP)
(72) Inventor: SHIMADA, Takahiro, Settu-Shi, Osaka 566-0045 (JP); FURUKAWA, Daisuke, Settu-Shi, Osaka 566-0045 (JP)
(74) Representative: Dr. Gassner & Partner mbB
(86) International application number: PCT/JP2020/018671
(87) International publication number: WO 2021/002095

(56) References cited:
- EP-A1- 3 037 079
- WO-A1-2017/131014
- WO-A1-2019/026971
- JP-A- 2002 113 800
- JP-A- 2005 515 020
- JP-A- 2006 297 077
- JP-A- 2008 516 806
- JP-A- 2017 099 855
- JP-A- 2018 502 627
- JP-A- H06 255 006
- JP-A- S58 155 855
- JP-B2- H07 100 067

## Description

### Technical Field

The present invention relates to a method of producing an absorbent body for an absorbent article, and more specifically, relates to a technology of producing an absorbent body containing an absorbent material.

### Background Art

An absorbent body used for absorbent articles such as disposable diapers, disposable shorts, sanitary napkins and incontinence pads contains a granular absorbent material capable of absorbing and holding liquid.

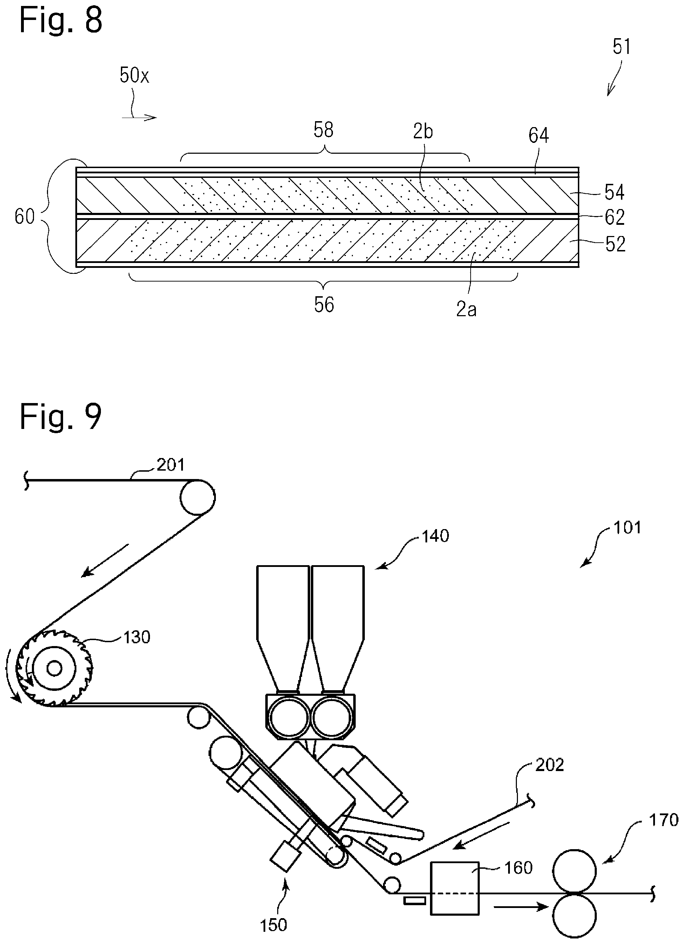

This absorbent body is produced, for example, by using an absorbent body producing apparatus 101 shown in Fig. 9. As shown in Fig. 9, one main surface side of a fiber sheet 201 being conveyed is nap-raised by a nap-raising device 130. Then, by using a scattering device 140, a granular absorbent material is scattered onto the nap-raised one main surface of the fiber sheet 201 while the other main surface of the fiber sheet 201 is supported with a planar conveyance surface, and by using a suction device 150, the other main surface of the fiber sheet 201 is suctioned through the conveyance surface to hold the scattered absorbent material on the fiber sheet 201. Then, a cover sheet 202 having a larger width than the fiber sheet 201 is laminated on the one main surface of the fiber sheet 201. Then, by using a folding device 160, the part of the cover sheet 202 protruding from the fiber sheet 201 is folded and overlapped with the other main surface of the fiber sheet 201. Then, by using a crimping device 170, the fiber sheet 201 and the cover sheet 202 are compressed in the thickness direction.

### Citation List

### Patent Literature

[Patent Literature 1] WO 2017/131014
[Patent Literature 2] WO2019/026971

### Summary of the Invention

### Problem to be Solved by the Invention

If the granular absorbent material scattered on the nap-raised fiber sheet 201 moves inside the fiber sheet 201 to be unevenly distributed due to vibrations during transport or the like, the liquid absorption decreases or rewet occurs. For this reason, it is desirable to prevent the scattered absorbent material from moving inside the fiber sheet 201.

Accordingly, it is considered to apply an adhesive to the nap-raised fiber sheet 201 and scatter the absorbent material thereon. However, when adhering to the applied adhesive, the scattered absorbent material is prevented from moving and cannot enter the inside of the fiber sheet. For this reason, a problem occurs in that a large amount of absorbent material remains on the absorbent-material-scattered side.

In view of such circumstances, a problem to be solved by the present invention is to provide a method of producing an absorbent body for an absorbent article, capable of preventing the absorbent material from moving in a state in which the granular absorbent material is infiltrated into the inside of the nap-raised fiber sheet.

### Means for Solving the Problem

To solve the above-mentioned problem, the present invention provides a method of producing an absorbent body for an absorbent article structured as follows:

The method of producing an absorbent body for an absorbent article is provided with: (i) a first step of supplying a base sheet having one main surface thereof applied with a first adhesive, to a conveyance device, and conveying the base sheet while suctioning another main surface of the base sheet through a conveyance surface of the conveyance device; (ii) a second step of overlapping, along the conveyance surface of the conveyance device, another main surface of a fiber sheet having one main surface thereof nap-raised with the one main surface of the base sheet applied with the first adhesive, thereby forming a first laminate where the fiber sheet is laminated on the base sheet; (iii) a third step of scattering a granular absorbent material capable of absorbing and holding liquid, onto the one main surface of the fiber sheet of the first laminate, and infiltrating the scattered absorbent material into an inside of the fiber sheet when the other main surface of the base sheet is suctioned through the conveyance surface of the conveyance device; (iv) a fourth step of overlapping one main surface of a cover sheet having the one main surface thereof applied with a second adhesive with the one main surface of the fiber sheet of the first laminate into the inside of which the absorbent material is infiltrated, thereby forming a second laminate where the cover sheet is laminated on the first laminate; and (v) a fifth step of compressing the second laminate in a thickness direction by using compression means, and spreading the first adhesive applied to the base sheet and the second adhesive applied to the cover sheet, in the inside of the fiber sheet of the second laminate.

According to the above-described method, since the first and second adhesives are spread in the inside of the fiber sheet after the granular absorbent material is infiltrated into the inside of the nap-raised fiber sheet, in a state in which the granular absorbent material is infiltrated into the inside of the nap-raised fiber sheet, the absorbent material can be prevented from moving.

Preferably, the compression means is a pair of rolls, and compresses the second laminate in the thickness direction when the second laminate passes between the pair of rolls.

In this case, the second laminate can be continuously compressed in the thickness direction with a simple structure.

Preferably, the first and second adhesives contain a thermoplastic resin. The compression means includes heating means for heating the second laminate.

In this case, by softening the first and second adhesives, the first and second adhesives can be effectively spread in the inside of the fiber sheet.

Preferably, the first and second adhesives contain a thermoplastic resin. The compression means is a pair of rolls, and compresses the second laminate in the thickness direction when the second laminate passes between the pair of rolls. At least one of the pair of rolls is heated.

In this case, the second laminate can be continuously compressed in the thickness direction with a simple structure. Moreover, by providing the pair of rolls with heating means and softening the first and second adhesives, the first and second adhesives can be effectively spread in the inside of the fiber sheet.

Preferably, the method of producing an absorbent material for an absorbent article is further provided with: (vi) a sixth step of supplying another base sheet having one main surface thereof applied with a third adhesive, to another conveyance device, and conveying the other base sheet while suctioning another main surface of the other base sheet through a conveyance surface of the other conveyance device; (vii) a seventh step of overlapping another main surface of another fiber sheet having one main surface thereof nap-raised with the one main surface of the other base sheet applied with the third adhesive, thereby forming a third laminate where the other fiber sheet is laminated on the other base sheet; (viii) an eighth step of scattering a granular other absorbent material capable of absorbing and holding liquid, onto the one main surface of the other fiber sheet of the third laminate, and infiltrating the scattered other absorbent material into an inside of the other fiber sheet when the other main surface of the other base sheet is suctioned through the conveyance surface of the other conveyance device; (ix) a ninth step of applying a fourth adhesive to the other main surface of the base sheet of the second laminate, and overlapping the other main surface of the base sheet of the second laminate applied with the fourth adhesive with the one main surface of the other fiber sheet of the third laminate into the inside of which the other absorbent material is infiltrated, thereby forming a fourth laminate where the second laminate is laminated on the third laminate; and (x) a tenth step of compressing the fourth laminate in the thickness direction by using other compression means, and spreading the third adhesive applied to the other base sheet and the fourth adhesive applied to the other main surface of the base sheet of the second laminate, in the inside of the other fiber sheet of the fourth laminate.

In this case, an absorbent body including two layers of fiber sheets can be produced. Since the third and fourth adhesives are spread inside the other fiber sheet after the granular other absorbent material is infiltrated into the inside of the nap-raised other fiber sheet, in a state in which the granular other absorbent material is infiltrated into the inside of the nap-raised other fiber sheet, the other absorbent material can be prevented from moving.

Since the distribution of the absorbent materials which is difficult in the absorbent body including only one layer of fiber sheet can be realized in the absorbent body including two layers of fiber sheets, the degree of freedom in design of the absorbent article is increased.

Preferably, when the fourth laminate is seen through in the thickness direction, (a) a plurality of first areas where the absorbent material is scattered so as to be continuous on the one main surface of the fiber sheet are disposed at intervals in a conveyance direction of the fourth laminate, (b) a plurality of second areas where the other absorbent material is scattered so as to be continuous on the one main surface of the other fiber sheet are disposed at intervals in the conveyance direction, (c) each of the first areas overlaps with only one of the second areas, respectively, and (d) the first areas are narrower than the second areas in the conveyance direction, or the second areas are narrower than the first areas in the conveyance direction.

In this case, the distribution of the absorbent materials in the thickness direction inside the absorbent body can be varied in the conveyance direction.

Moreover, the present invention provides a method of producing an absorbent article as claimed in claim 7.

The absorbent article includes the absorbent body produced by the above-described method of producing an absorbent body for an absorbent article, (A) when seen through in the thickness direction, the first areas are narrower than the second areas in the conveyance direction and the fiber sheet is disposed on a skin surface side of the other fiber sheet, or (B) when seen through in the thickness direction, the second areas are narrower than the first areas in the conveyance direction and the other fiber sheet is disposed on the skin surface side of the fiber sheet.

In this case, since the absorbent materials are disposed more widely on the non-skin surface side than on the skin surface side, rewet is less likely to occur than when the absorbent materials are disposed more widely on the skin surface side than on the non-skin surface side.

### Effects of the Invention

According to the present invention, in a state in which the granular absorbent material is infiltrated into the inside of the nap-raised fiber sheet, the absorbent material can be prevented from moving.

### Brief Description of the Drawings

Fig. 1 is a schematic view of an absorbent body producing apparatus (first embodiment).
Fig. 2 is a relevant part enlarged view of the absorbent body producing apparatus (first embodiment).
Figs. 3A and 3B are schematic views of an absorbent body (first embodiment).
Fig. 4 is a schematic view of an absorbent body producing apparatus (first modification).
Fig. 5 is a schematic view of an absorbent body producing apparatus (second embodiment).
Figs. 6A and 6B are schematic views of an absorbent body (second embodiment).
Figs. 7A to 7C are schematic views of a second to a fourth laminate seen through in the thickness direction (second embodiment).
Fig. 8 is a schematic view of the absorbent body cut vertically in the width direction (second embodiment).
Fig. 9 is a schematic view showing the absorbent body producing apparatus (first conventional example).

### Mode for Carrying out the Invention

Hereinafter, embodiments of the present invention will be described with reference to the drawings.

### <First embodiment>

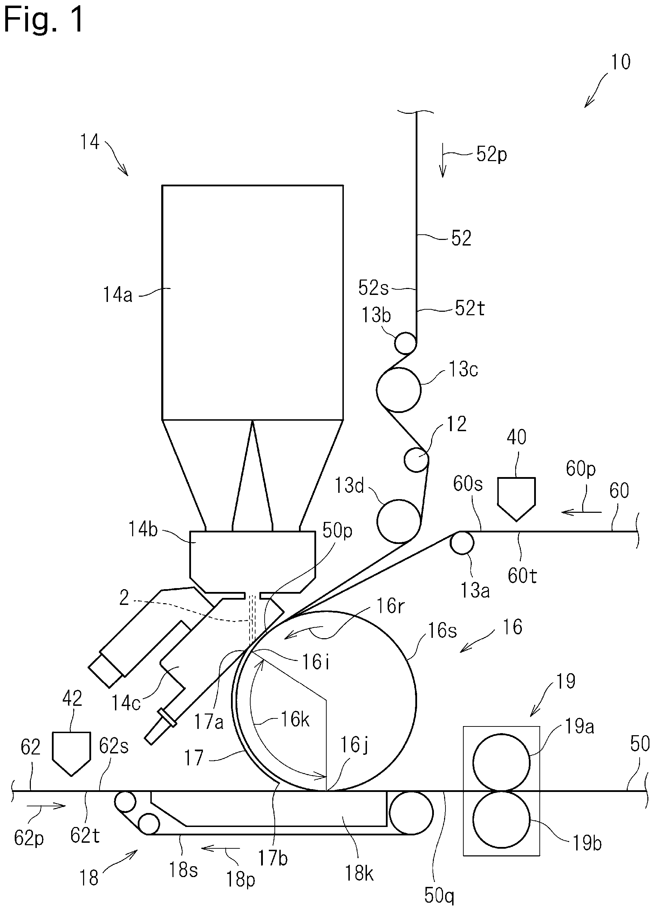

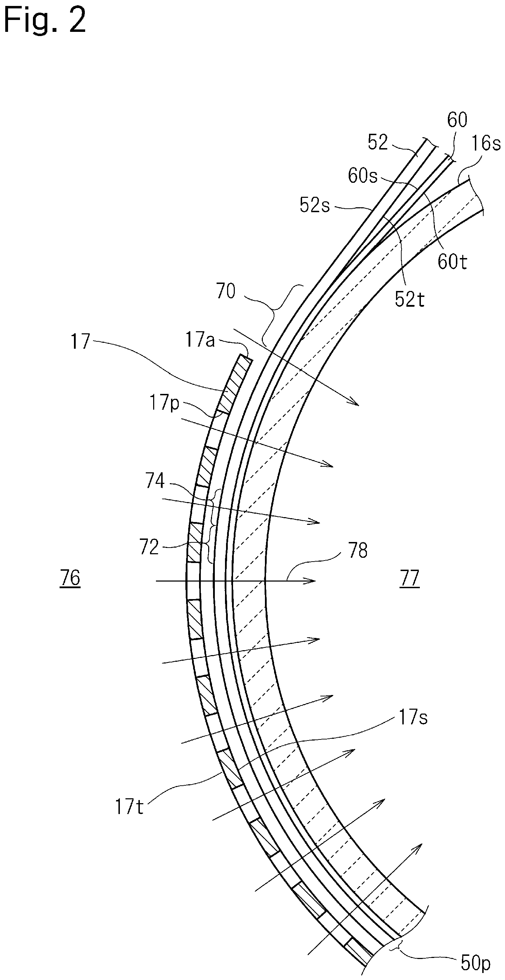

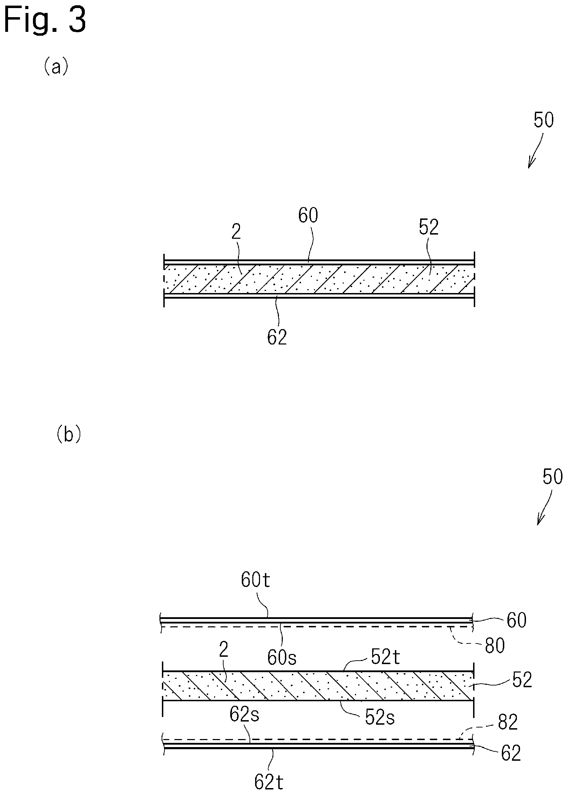

A method of producing an absorbent body for an absorbent article according to the first embodiment will be described with reference to Figs. 1 to 3B. Fig. 1 is a schematic view of an absorbent body producing apparatus 10 used in the first embodiment. Fig. 2 is a relevant part enlarged view of the absorbent body producing apparatus 10. Figs. 3A and 3B are schematic views of an absorbent body 50 cut vertically to a conveyance direction. Fig. 3A is an assembled view, and Fig. 3B is a disassembled view.

As shown in Fig. 1, in the absorbent body producing apparatus 10, after a breathable continuous base sheet 60 and fiber sheet 52 supplied to a first conveyance device 16 and a continuous cover sheet 62 supplied to a second conveyance device 18 are overlapped with each other, they are compressed in the thickness direction by a compression device 19 and before the cover sheet 62 is overlapped, a granular absorbent material 2 capable of absorbing and holding liquid is scattered from a scattering device 14 onto one main surface 52s of the fiber sheet 52 previously nap-raised by a nap-raising device 12, thereby continuously producing the absorbent body 50. The absorbent material 2 is, for example, SAP (super absorbent polymer) .

In the first conveyance device 16, a breathable cylindrical conveyance surface 16a having a mesh structure or the like rotates in the direction indicated by the arrow 16r, and the base sheet 60 is wound on the conveyance surface 16s. The first conveyance device 16 has inside a space (vacuum chamber) being negative in pressure, and air is suctioned inward from the conveyance surface 16s through the conveyance surface 16s in a section 16k (hereinafter, referred to as suction section 16k) between a first position 16i where the base sheet 60 has moved by a predetermined distance after being wound and a second position 16j where the base sheet 60 separates from the conveyance surface 16s.

The base sheet 60 is conveyed in the direction indicated by the arrow 60p, and a first adhesive 80 such as hot melt adhesive (see Fig. 3B) is applied to one main surface 60s thereof by a first applying device 40 in such a manner that the breathability is maintained. Then, after passing a guide roll 13a, the base sheet 60 is supplied to the first conveyance device 16 so that the other main surface 60t thereof is in contact with the conveyance surface 16s of the first conveyance device 16 (see Fig. 2), and is wound on the conveyance surface 16s of the first conveyance device 16. The base sheet 60 is suctioned through the conveyance surface 16s in the suction section 16k, and moves together with the conveyance surface 16s. The base sheet 60 is, for example, a sheet of tissue paper.

The fiber sheet 52 is conveyed in the direction indicated by the arrow 52p, is wound on the nap-raising device 12 by guide rolls 13b to 13d, and has one main surface 52s side thereof nap-raised by the nap-raising device 12. Then, the fiber sheet 52 is supplied to the first conveyance device 16 in such a manner that the other main surface 52t is overlapped with the one main surface 60s of the base sheet 60 wound on the conveyance surface 16s of the first conveyance device 16 (see Fig. 2), a first laminate 50p where the fiber sheet 52 is laminated on the base sheet 60 is formed, and the fiber sheet 52 moves together with the base sheet 60. The fiber sheet 52 is, for example, an air-through nonwoven fabric. The nap-raising device 12 is, for example, a tiller in which a plurality of blade edges scratch the one main surface 52s of the fiber sheet 52 to nap-raise the one main surface 52s side so as to be bulky. The nap-raising device 12 is unnecessary when the fiber sheet 52 having the one main surface 52s side thereof previously nap-raised is used.

The scattering device 14 scatters the absorbent material 2 onto the nap-raised one main surface 52s of the fiber sheet 52 of the first laminate 50p. The absorbent material 2 is scattered onto a scatter area 70 (see Fig. 2) on the upstream side in the conveyance direction of the first position 16i where suction is started. The scattering device 14 scatters the absorbent material 2 onto the one main surface 52s of the fiber sheet 52, for example, by mixing a plurality of kinds of absorbent materials stored in a storage 14a by a mixing device 14b and discharging the mixed absorbent material 2 from a non-illustrated scatter port of an opening and closing device 14c. The opening and closing device 14c opens and closes the scatter port to thereby scatter the absorbent material 2 intermittently, that is, in such a manner as to continue at intervals in the conveyance direction of the fiber sheet 52.

A member 17 in the form of a plate (hereinafter, referred to as plate-like member 17) is disposed so as to face, with a space in between, the one main surface 52s of the fiber sheet 52 of the first laminate 50p scattered with the absorbent material 2 and being conveyed in the suction section 16k. One end 17a of the plate-like member 17 adjoins the non-illustrated scatter port of the scattering device 14, and the other end 17b of the plate-like member 17 adjoins the second conveyance device 18.

In the second conveyance device 18, a breathable endless belt 18s having a mesh structure or the like circulates in the direction indicated by the arrow 18p, and a vacuum box 18k is disposed inside of the endless belt 18s.

The cover sheet 62 is conveyed in the direction indicated by the arrow 62p, and a second adhesive 82 such as hot melt adhesive (see Fig. 3B) is applied to one main surface 62s thereof by a second applying device 42; then, the cover sheet 62 is supplied to the second conveyance device 18 in such a manner that the other main surface 62t is in contact with the endless belt 18s of the second conveyance device 18, and moves together with the endless belt 18s while being suctioned through the endless belt 18s. The cover sheet 62 is, for example, a sheet of tissue paper.

The first conveyance device 16 and the second conveyance device 18 are disposed close to each other, and the one main surface 52s of the fiber sheet 52 of the first laminate 50p being conveyed by the first conveyance device 16 is overlapped with the one main surface 62s, applied with the second adhesive 82 (see Fig. 3B), of the cover sheet 62 being conveyed by the second conveyance device 18, whereby a second laminate 50q where the cover sheet 62 is laminated on the first laminate 50p is formed.

The second laminate 50q, that is, the laminated base sheet 60, fiber sheet 52 and cover sheet 62 pass between a pair of rolls 19a and 19b of the compression device 19 to be compressed in the thickness direction, so that the first adhesive 80 (see Fig. 3B) applied to the base sheet 60 and the second adhesive 82 (see Fig. 3B) applied to the cover sheet 62 spread in the inside of the fiber sheet 52 of the second laminate 50q. This forms the continuous absorbent body 50 where the absorbent material 2 inside the fiber sheet 52 is fixed. Although not shown, the continuous absorbent body 50 is conveyed to a post-process to be cut into pieces, and then, disposed in appropriate positions of an absorbent article.

Although the second laminate 50q may be compressed in the thickness direction by using compression means other than the pair of rolls 19a and 19b, the use of the pair of rolls 19a and 19b enables the second laminate 50q to be continuously compressed in the thickness direction with a simple structure.

It is preferable for the compression means to include heating means for heating the second laminate 50q. For example, at least one of the pair of rolls 19a and 19b is heated by using the first and second adhesives 80 and 82 containing a thermoplastic resin such as hot melt adhesive. In this case, the second laminate 50q is heated to soften the first and second adhesives 80 and 82, whereby the first and second adhesives 80 and 82 can be effectively spread in the inside of the fiber sheet 52.

The first and second adhesives 80 and 82 may be either the same adhesive or different adhesives. Moreover, the first and second adhesives 80 and 82 may be other than adhesives containing a thermoplastic resin.

Next, the plate-like member 17 will be further described. As shown in Fig. 2, one main surface 17s of the plate-like member 17 faces, with a space in between, the one main surface 52s of the fiber sheet 52 of the first laminate 50p scattered with the absorbent material 2 and being conveyed in the suction section 16k. The plate-like member 17 has a plurality of through holes 17p passing through between the main surfaces 17s and 17t. Since the base sheet 60 is suctioned through the conveyance surface 16s, as indicated by the arrows 78, the air flow is caused that passes through the through holes 17p of the plate-like member 17 and penetrates the fiber sheet 52 to move inward from the conveyance surface 16s.

Compared with when no plate-like member 17 is provided, in a second facing area 74 facing the through holes 17p of the plate-like member 17, the air flow is high in flow speed until it impinges on the one main surface 52s of the fiber sheet 52, and high in dynamic pressure on the one main surface 52s side of the fiber sheet 52. For this reason, compared with when no plate-like member 17 is provided, the difference in atmospheric pressure, that is, the difference in static pressure is large between the conveyance surface 16s side (in Fig. 2, the area indicated by reference numeral 76) and the opposite side (in Fig. 2, the area indicated by reference numeral 77) of the fiber sheet 52.

The provision of the plate-like member 17 makes it possible to enhance the air flow passing through the fiber sheet 52 to move toward the conveyance surface 16s to increase the force by which the granular absorbent material 2 scattered onto the one main surface 52s of the fiber sheet 52 is suctioned toward the other main surface 52t of the fiber sheet 52.

Moreover, it is possible to make the air flow weak in a first facing area 72 facing the part of the plate-like member 17 where no through holes 17p are formed and make the air flow strong in the second facing area 74 facing the through holes 17p of the plate-like member 17. In this case, the fiber sheet 52 passes the first facing area 72 where the air flow is weak and the second facing area 74 where the air flow is strong, so that shaking of each fiber of the fiber sheet 52 becomes fierce and changes in the flow direction and strength of the air become drastic inside the fiber sheet 52. For this reason, the absorbent material 2 scattered onto the one main surface 52s of the fiber sheet 52 is readily released even if entangled with the fibers of the fiber sheet 52, so that it easily moves inside the fiber sheet 52 toward the other main surface 52t of the fiber sheet 52.

Although the granular absorbent material 2 can be infiltrated into the inside of the fiber sheet 52 of the first laminate 50p without the provision of the plate-like member 17, the provision of the plate-like member 17 enables a larger amount of granular absorbent material 2 to be infiltrated into the inside of the fiber sheet 52 from the nap-raised one main surface 52s of the fiber sheet 52.

In the absorbent body 50, as shown in Figs. 3A and 3B, the one main surface 60s of the base sheet 60 and the other main surface 52t of the fiber sheet 52 are bonded together by the first adhesive 80. The one main surface 62s of the cover sheet 62 and the one main surface 52s of the fiber sheet 52 are bonded together by the second adhesive 82. Inside the fiber sheet 52, the absorbent material 2 is disposed. While it is preferable to use the same adhesive as the first and second adhesives 80 and 82, adhesives different in ingredients may be used.

When the absorbent body 50 is used for the absorbent article, the absorbent article is formed as follows: The one main surface 52s of the fiber sheet 52 is disposed on the skin surface side (that is, the side close to the user's skin in a state in which the absorbent article is being used) and the other main surface 52t of the fiber sheet 52 is disposed on the non-skin surface side (that is, the side farther from the user's skin in a state in which the absorbent article is being used).

In the absorbent body 50, as mentioned previously, a larger amount of absorbent material 2 scattered onto the nap-raised one main surface 52s of the fiber sheet 52 can be infiltrated into the inside of the fiber sheet 52. For this reason, the amount of absorbent material 2 disposed on the one main surface 52s side of the fiber sheet 52 can be made small. Although a large mount of absorbent material 2 disposed on the skin surface side gives a rough touch, the rough touch can be suppressed because the amount of absorbent material 2 disposed on the skin surface side can be made small.

A method of producing an absorbent material for an absorbent article according to the first embodiment for producing the absorbent body 50 by using the above-described absorbent body producing apparatus 10 is provided with the following first to five steps:
(i) At the first step, the base sheet 60 having the one main surface 60s thereof applied with the first adhesive 80 is supplied to the first conveyance device 16 and conveyed while the other main surface 60t of the base sheet 60 is suctioned through the conveyance surface 16s of the first conveyance device 16. (ii) At the second step, the other main surface 52t of the fiber sheet 52 having the one main surface 52s thereof nap-raised is overlapped with the one main surface 60s of the base sheet 60 applied with the first adhesive 80, thereby forming the first laminate 50p where the fiber sheet 52 is laminated on the base sheet 60. (iii) At the third step, the granular absorbent material 2 capable of absorbing and holding liquid is scattered onto the one main surface 52s of the fiber sheet 52 of the first laminate 50p, and the scattered absorbent material 2 is infiltrated into the inside of the fiber sheet 52 when the other main surface 60t of the base sheet 60 is suctioned through the conveyance surface 16s of the first conveyance device 16. (iv) At the fourth step, the one main surface 62s of the cover sheet 62 having the one main surface 62s thereof applied with the second adhesive 82 is overlapped with the one main surface 52s of the fiber sheet 52 of the first laminate 50p into the inside of which the absorbent material 2 is infiltrated, thereby forming the second laminate 50q where the cover sheet 62 is laminated on the first laminate 50p. (v) At the fifth step, the second laminate 50q is compressed in the thickness direction by using the compression means (compression device 19), so that the first adhesive 80 applied to the base sheet 60 and the second adhesive 82 applied to the cover sheet 62 are spread in the inside of the fiber sheet 52 of the second laminate 50q.

If the first adhesive 80 is applied to the one main surface 52s of the fiber sheet 52 and the granular absorbent material 2 is scattered thereon to unlike the above-described method of the first embodiment, since the absorbent material 2 is bonded to the first adhesive 80, the absorbent material 2 is not easily infiltrated into the inside of the fiber sheet 52. On the contrary, according to the above-described method of the first embodiment, since the first adhesive 80 is not directly applied to the one main surface 52s of the fiber sheet 52 onto which the absorbent material 2 is scattered, the absorbent material 2 scattered onto the one main surface 52s of the fiber sheet 52 is easily infiltrated into the inside of the fiber sheet 52. For this reason, the absorbent material 2 can be broadly distributed inside the fiber sheet 52.

The second laminate 50q in a state in which the absorbent material 2 is broadly distributed inside the fiber sheet 52 is compressed to spread the first adhesive 80 applied to the base sheet 60 and the second adhesive 82 applied to the cover sheet 62 inside the fiber sheet 52, which makes it possible to fix the absorbent material 2 distributed inside the fiber sheet 52 inside the fiber sheet 52.

Consequently, in a state in which the granular absorbent material 2 is infiltrated into the inside of the nap-raised fiber sheet 52, the absorbent material 2 can be prevented from moving.

### <First modification>

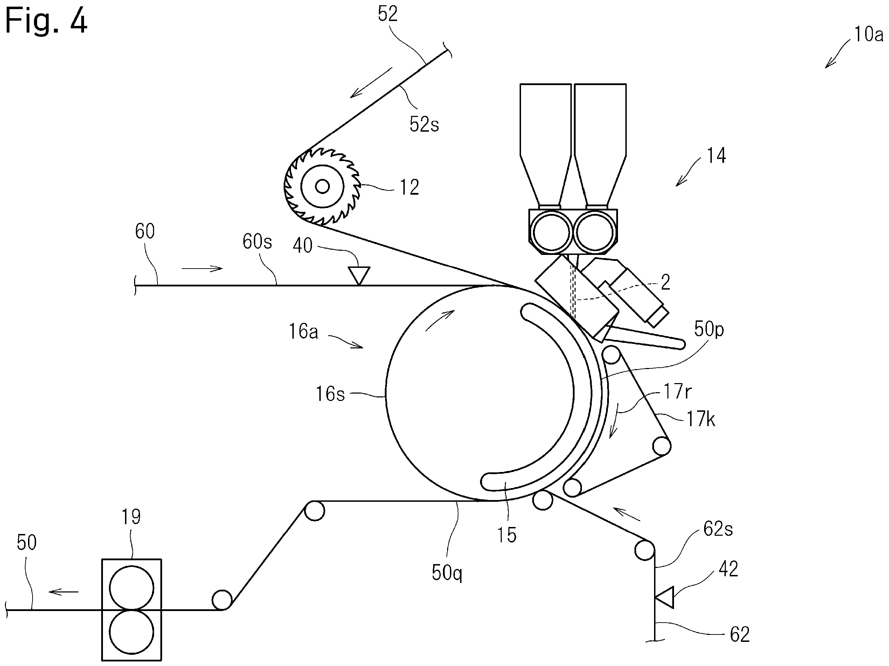

Fig. 4 is a schematic view of an absorbent body producing apparatus 10a of the first modification in which a belt-like member 17k is used instead of the plate-like member 17. As shown in Fig. 4, the absorbent body producing apparatus 10a is structured substantially similarly to the absorbent body producing apparatus 10 used in the first embodiment. Hereinafter, differences from the first embodiment will be mainly described, and parts of the same structure as those of the first embodiment are denoted by the same reference numerals.

As in the first embodiment, after a first adhesive such as hot melt adhesive is applied to the one main surface 60s by the first applying device 40 in such a manner that the breathability is maintained, the base sheet 60 is wound on the breathable cylindrical conveyance surface 16s of a first conveyance device 16a. After the one main surface 52s side is nap-raised by the nap-raising device 12, the fiber sheet 52 is overlapped with the one main surface 60s of the base sheet 60 where the first adhesive is applied. This forms the first laminate 50p. In the first laminate 50p, the absorbent material 2 supplied from the scattering device 14 is scattered onto the one main surface 52s of the fiber sheet 52.

Inside the first conveyance device 16a, a vacuum box 15 is provided so that the base sheet 60 is suctioned through the conveyance surface 16s over the entire section where the base sheet 60 is wound. Unlike the first embodiment, the suction section is the entire section where the base sheet 60 is wound on the conveyance surface 16s, and the absorbent material 2 is scattered in the middle of the suction section.

After the second adhesive such as hot melt adhesive is applied to the one main surface 62s by the second applying device 42, the cover sheet 62 is overlapped with the one main surface 52s of the fiber sheet 52 of the first laminate 50p scattered with the absorbent material 2. This forms the second laminate 50q. Unlike the first embodiment, the second conveyance device 18 is absent, and the cover sheet 62 and the second laminate 50q are conveyed by the first conveyance device 16a.

The second laminate 50q is discharged from the first conveyance device 16a and is then compressed in the thickness direction by means of the compression device 19, so that the first and second adhesives spread in the inside of the fiber sheet 52. This forms the continuous absorbent body 50 where the absorbent material 2 is fixed.

The belt-like member 17k is a breathable endless belt, and is structured so as to circulate in the direction indicated by the arrow 17r. For example, the belt-like member 17k is formed so as to have a mesh structure or the like, or through holes, slits or the like are formed on the belt-like member 17k.

While the belt-like member 17k and the fiber sheet 52 of the first laminate 50p wound on the conveyance surface 16s of the first conveyance device 16a are shown as being separated from each other in Fig. 4, the belt-like member 17k moves together with the fiber sheet 52 while being in contact with the one main surface 52s of the fiber sheet 52 scattered with the absorbent material 2. That is, the first laminate 50p where the fiber sheet 52 and the base sheet 60 are laminated is conveyed while being sandwiched between the conveyance surface 16s and the belt-like member 17k and at this time, is suctioned through the conveyance surface 16s so that air flows from the belt-like member 17k side to the conveyance surface 16s side.

By appropriately structuring the belt-like member 17k, the air flow penetrating the fiber sheet 52 to move toward the conveyance surface 16s is suppressed, so that the atmospheric pressure difference between the conveyance surface 16s side and the opposite side of the fiber sheet 52 can be made large. This enables a larger amount of granular absorbent material 2 to be infiltrated into the inside of the fiber sheet 52 from the nap-raised one main surface 52s of the fiber sheet 52.

Consequently, as in the first embodiment, in a state in which the granular absorbent material 2 is infiltrated into the inside of the nap-raised fiber sheet 52, the absorbent material 2 can be prevented from moving.

### <Second embodiment>

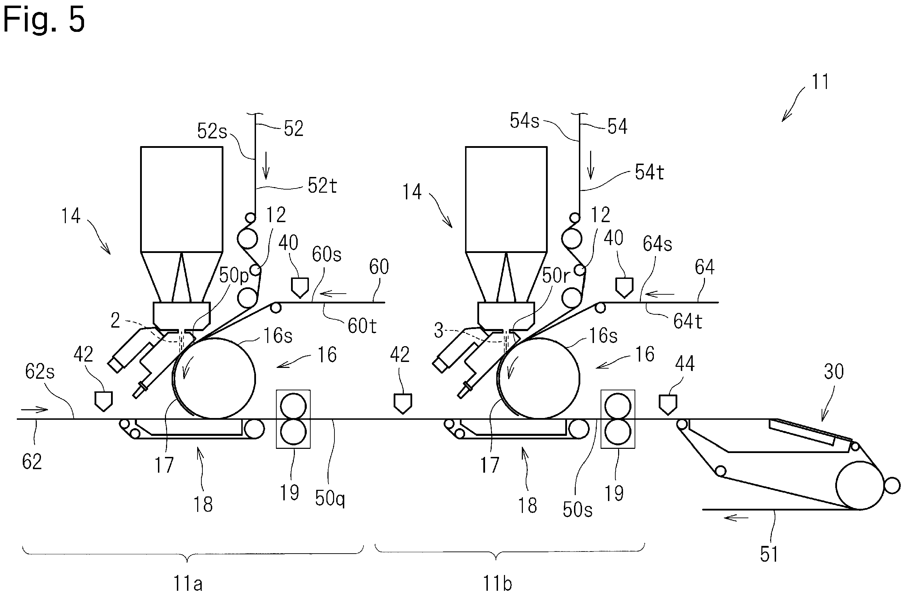

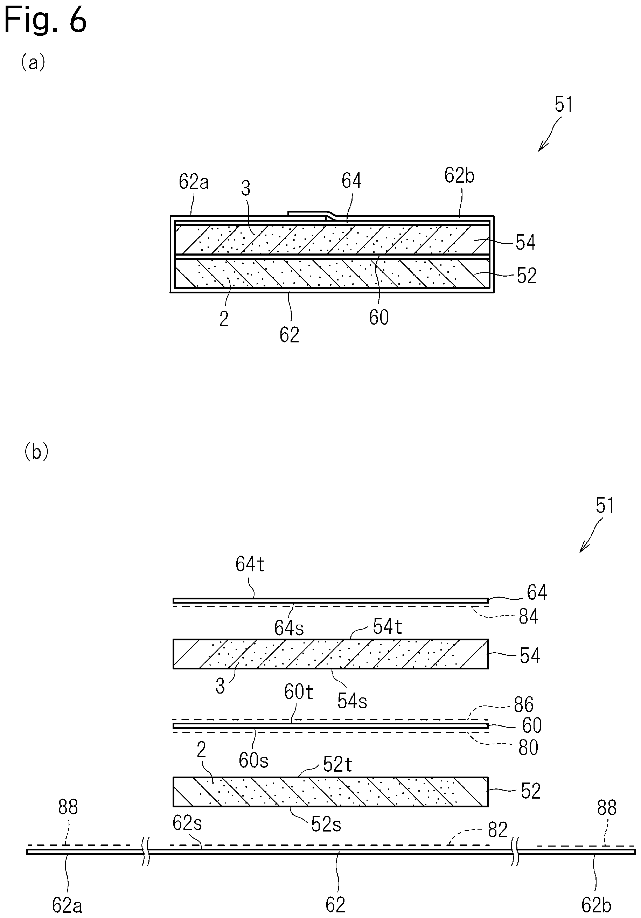

A second embodiment in which an absorbent body 51 including two layers of fiber sheets 52 and 54 is produced will be described with reference to Figs. 5 to 8. Fig. 5 is a schematic view of an absorbent body producing apparatus 11 used in the second embodiment. Figs. 6A and 6B are schematic views of the absorbent body 51 cut vertically to a conveyance direction. Fig. 6A is an assembled view, and Fig. 6B is a disassembled view.

As shown in Fig. 5, the absorbent body producing apparatus 11 is provided with: first and second apparatuses 11a and 11b having the same structure as the absorbent body producing apparatus 10 of the first embodiment; a third applying device 44; and a folding device 30.

The first apparatus 11a continuously produces, as in the first embodiment, the second laminate 50q in which the continuous base sheet 60 (hereinafter, referred to also as intermediate sheet 60), the continuous fiber sheet 52 and the continuous cover sheet 62 are laminated. The second laminate 50q is compressed in the thickness direction by means of the compression device 19.

The second apparatus 11b continuously produces, substantially as in the first embodiment, a fourth laminate 50s in which a continuous other base sheet 64, a continuous other fiber sheet 54 and the continuous second laminate 50q are laminated. The fourth laminate 50s is compressed in the thickness direction by means of the compression device 19.

Specifically, in the second apparatus 11b, the other fiber sheet 54 has one main surface 54s side thereof nap-raised by the nap-raising device 12 before supplied to the first conveyance device 16. The other base sheet 64 has breathability, and before it is supplied to the first conveyance device 16, a third adhesive 84 such as hot melt adhesive (see Fig. 6) is applied to one main surface 64s by the first applying device 40 in such a manner that the breathability is maintained. The other main surface 64t of the other base sheet 64 is suctioned through the conveyance surface 16s of the first conveyance device 16. The other main surface 54t of the other fiber sheet 54 and the one main surface 64s of the other base sheet 64 are overlapped with each other to form a third laminate 50r where the other fiber sheet 54 is laminated on the other base sheet 64.

In the second laminate 50q formed by the first apparatus 11a, after a fourth adhesive 86 such as hot melt adhesive (see Fig. 6B) is applied to the other main surface 60t of the intermediate sheet 60 of the second laminate 50q by the second applying device 42, the other main surface 60t of the intermediate sheet 60 of the second laminate 50q is overlapped with the one main surface 54s of the other fiber sheet 54 of the third laminate 50r. This forms the fourth laminate 50s where the second laminate 50q is laminated on the third laminate 50r.

As shown in Figs. 6A and 6B, the cover sheet 62 is larger in the dimension in the width direction (a direction vertical to the conveyance direction and the thickness direction) than the other sheets 52, 54, 60 and 64, and is laminated so as to protrude from both sides in the width direction of the other sheets 52, 54, 60 and 64.

As shown in Figs. 5, 6A and 6B, in the third applying device 44, a fifth adhesive 88 such as hot melt adhesive is applied to the one main surface 62s of parts 62a and 62b on both sides in the width direction of the cover sheet 62. The folding device 30 folds the parts 62a and 62b on both sides in the width direction of the cover sheet 62 applied with the fifth adhesive 88 and overlaps them with the other base sheet 64. This forms the continuous absorbent body 51 structured so as to be wrapped with the cover sheet 62.

As shown in Figs. 6A and 6B, in the absorbent body 51, the one main surface 60s of the intermediate sheet 60 and the other main surface 52t of the fiber sheet 52 are bonded together by the first adhesive 80 and the one main surface 62s of the cover sheet 62 and the one main surface 52s of the fiber sheet 52 are bonded together by the second adhesive 82. Moreover, the one main surface 64s of the other base sheet 64 and the other main surface 54t of the other fiber sheet 54 are bonded together by the third adhesive 84, and the other main surface 60t of the intermediate sheet 60 and the one main surface 54s of the other fiber sheet 54 are bonded together by the fourth adhesive 86. The one main surface 62s of parts 62a and 62b on both sides in the width direction of the cover sheet 62 and the other main surface 64t of the other base sheet 64 are bonded together by the fifth adhesive 88. The absorbent material 2 is disposed inside the fiber sheet 52, and an other absorbent material 3 is disposed inside the other fiber sheet 54. While it is preferable to use the same adhesive as the first to fifth adhesives 80, 82, 84, 86 and 88, adhesives different in ingredients may be used.

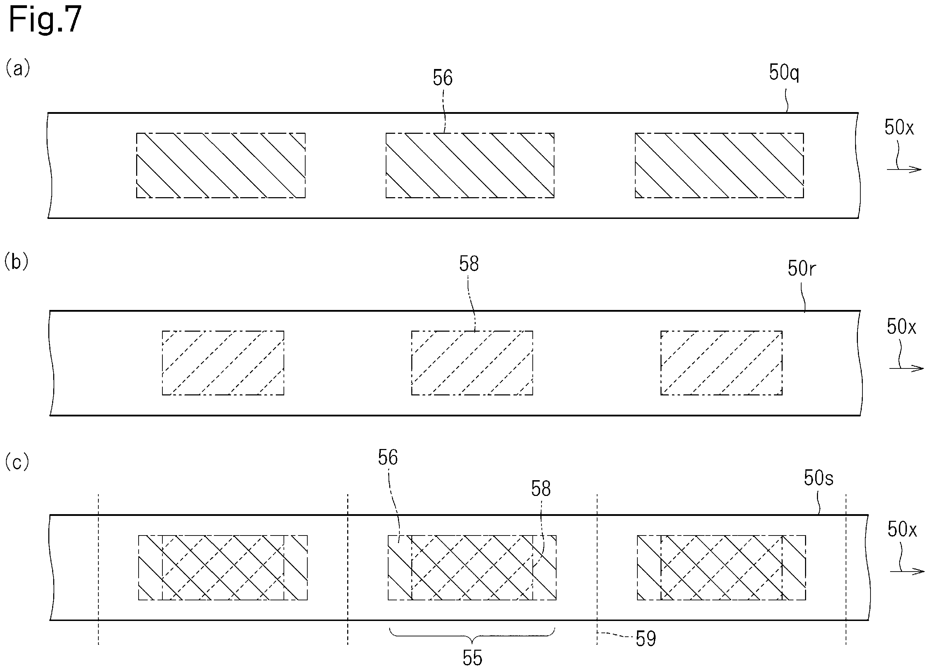

Figs. 7A to 7C are schematic views of the second to fourth laminates 50q to 50s seen through in the thickness direction. In Figs. 7A to 7C, the direction indicated by the arrow 50x is the conveyance direction.

As shown in Fig. 7A, when the second laminate 50q is seen through in the thickness direction, a plurality of first areas 56 where the absorbent material 2 is scattered so as to be continuous on the one main surface 52s of the fiber sheet 52 are disposed at intervals in the conveyance direction.

As shown in Fig. 7B, when the third laminate 50r is seen through in the thickness direction, a plurality of second areas 58 where the other absorbent material 3 is scattered so as to be continuous on the one main surface 54s of the other fiber sheet 54 are disposed at intervals in the conveyance direction.

As shown in Fig. 7C, when the fourth laminate 50s is seen through in the thickness direction, each of the first areas 56 overlaps with only one of the second areas 58, respectively, and a plurality of continuous areas 55 where one of the first areas 56 and one of the second areas 58 overlap with each other, respectively, are disposed at intervals in the conveyance direction. The fourth laminate 50s is, as indicated by the broken lines 59, cut between the adjoining continuous areas 55 into pieces, and disposed on a non-illustrated absorbent article.

When seen through in the thickness direction, the first areas 56 and the second areas 58 may be either the same or different from each other in area and shape, and the first areas 56 and the second areas 58 may be situated so as to completely coincide with each other or may be situated so as to be shifted from each other.

In the absorbent body 51 where one of the first area 56 and the second area 58 is narrower than the other thereof in the conveyance direction as shown in Fig. 7C, the distribution of the absorbent materials 2 and 3 in the thickness direction can be varied in the conveyance direction.

Fig. 8 is a schematic view of the absorbent body 51 cut vertically in the width direction. When the second area 58 is narrower than the first area 56 in the conveyance direction as shown in Fig. 8, it is desirable to dispose the absorbent body 51 on the absorbent article so that the other fiber sheet 54 is on the skin side of the fiber sheet 52. On the other hand, although not shown, when the first area 56 is narrower than the second area 58 in the conveyance direction, it is desirable to dispose the absorbent body 51 on the absorbent article so that the fiber sheet 52 is on the skin side of the other fiber sheet 54.

When the absorbent body 51 is disposed as described above, since the absorbent materials 2 and 3 are disposed more widely on the non-skin surface side than on the skin surface side, rewet is less likely to occur than when the absorbent materials 2 and 3 are disposed more widely on the skin surface side than on the non-skin surface side.

According to the method of producing an absorbent body for an absorbent article according to the second embodiment for producing the absorbent body 51 by using the above-described absorbent body producing apparatus 11, the above-described first to fifth steps are performed by using the first apparatus 11a and the following sixth to tenth steps are performed by using the second apparatus 11b:
(vi) At the sixth step, the other base sheet 64 having the one main surface 64s thereof applied with the third adhesive 84 is supplied to the other conveyance device (the first conveyance device 16 of the second apparatus 11b) and conveyed while the other main surface 64t of the other base sheet 64 is suctioned through the conveyance surface of the other conveyance device (the conveyance surface 16s of the first conveyance device 16 of the second apparatus 11b). (vii) At the seventh step, the other main surface 54t of the other fiber sheet 54 having the one main surface 54s thereof nap-raised is overlapped with the one main surface 64s of the other base sheet 64 applied with the third adhesive 84, thereby forming the third laminate 50r where the other fiber sheet 54 is laminated on the other base sheet 64. (viii) At the eighth step, the granular other absorbent material 3 capable of absorbing and holding liquid is scattered onto the one main surface 54s of the fiber sheet 54 of the third laminate 50r, and the scattered absorbent material 3 is infiltrated into the inside of the other fiber sheet 54 when the other main surface 64t of the other base sheet 64 is suctioned through the conveyance surface of the other conveyance device (the conveyance surface 16s of the first conveyance device 16 of the second apparatus 11b). (ix) At the ninth step, the fourth adhesive 86 is applied to the other main surface 60t of the base sheet 60 of the second laminate 50q, and the other main surface 60t of the base sheet 60 of the second laminate 50q applied with the fourth adhesive 86 is overlapped with the one main surface 54s of the other fiber sheet 54 of the third laminate 50r into the inside of which the other absorbent material 3 is infiltrated, thereby forming the fourth laminate 50s where the second laminate 50q is laminated on the third laminate 50r. (x) At the tenth step, the fourth laminate 50s is compressed in the thickness direction by using the other compression means (the compression device 19 of the second apparatus 11b), so that the third adhesive 84 applied to the other base sheet 64 and the fourth adhesive 86 applied to the base sheet 60t of the base sheet 60 of the second laminate 50q are spread in the inside of the other fiber sheet 54 of the fourth laminate 50s.

In the second embodiment, as in the first embodiment, by the first to fifth steps, in a state in which the granular absorbent material 2 is infiltrated into the inside of the nap-raised fiber sheet 52, the absorbent material 2 can be prevented from moving. Moreover, by the sixth to tenth steps, in a state in which the granular other absorbent material 3 is infiltrated into the inside of the nap-raised other fiber sheet 54, the other absorbent material 3 can be prevented from moving.

By the above-described method of the second embodiment, the absorbent body 51 including the two layers of fiber sheets 52 and 54 can be produced from the fourth laminate 50s. In the absorbent body 51 including the two layers of the fiber sheets 52 and 54, the combination of the distribution of the absorbent material 2 in the fiber sheet 52 of the first layer and the distribution of the other absorbent material 3 in the other fiber sheet 54 of the second layer enables the realization of the distribution of the absorbent materials 2 and 3 which is difficult in the absorbent body 50 including only one layer of fiber sheet 52 as in the first embodiment. For this reason, the use of the absorbent body 51 including the two layers of fiber sheets 52 and 54 increases the degree of freedom in design of the absorbent article.

### <Summary>

As described above, by compressing the laminates 50q; 50q and 50r in the thickness direction and spreading the applied adhesives 80, 82; 80, 82, 84 and 86 in the inside of the nap-raised fiber sheets 52; 52 and 54, in a state in which the granular absorbent materials 2; 2 and 3 are infiltrated into the inside of the nap-raised fiber sheets 52; 52 and 54, the absorbent materials 2; 2 and 3 can be prevented from moving.

The present invention is not limited to the above-described embodiments and may be variously modified when carried out.

For example, while a case is shown in which the other main surface side of the fiber sheet is suctioned through the cylindrical conveyance surface of the conveyance device, it is necessary only that the conveyance surface of the conveyance device is structured appropriately; for example, the conveyance surface of the conveyance device may be planar.

### Description of Reference Numerals

- 2: Absorbent material
- 3: Other absorbent material
- 16, 16a: First conveyance device (conveyance device)
- 16s: Conveyance surface
- 19: Compression device (compression means)
- 19a, 19b: Rolls
- 50: Absorbent body
- 50p: First laminate
- 50q: Second laminate
- 50r: Third laminate
- 50s: Fourth laminate
- 51: Absorbent body
- 52: Fiber sheet
- 52s: One main surface
- 52t: Other main surface
- 54: Other fiber sheet
- 54s: One main surface
- 54t: Other main surface
- 56: First area
- 58: Second area
- 60: Base sheet
- 60s: One main surface
- 60t: Other main surface
- 62: Cover sheet
- 62s: One main surface
- 62t: Other main surface
- 64: Other base sheet
- 64s: One main surface
- 64t: Other main surface
- 80: First adhesive
- 82: Second adhesive
- 84: Third adhesive
- 86: Fourth adhesive
- 88: Fifth adhesive

## Claims

1. A method of producing an absorbent body (50, 51) for an absorbent article, the method comprising:
a first step of supplying a base sheet (60) having one main surface (60s) thereof applied with a first adhesive (80), to a conveyance device (16), and conveying the base sheet (60) while suctioning another main surface (60t) of the base sheet (60) through a conveyance surface of the conveyance device (16);
a second step of overlapping, along the conveyance surface (16s) of the conveyance device (16), another main surface (52t) of a fiber sheet (52) having one main surface (52s) thereof nap-raised with the one main surface (60s) of the base sheet (60) applied with the first adhesive (80), thereby forming a first laminate (50p) where the fiber sheet (52) is laminated on the base sheet (60);
a third step of scattering a granular absorbent material (2) capable of absorbing and holding liquid, onto the one main surface (52s) of the fiber sheet (52) of the first laminate (50p), and infiltrating the scattered absorbent material (2) into an inside of the fiber sheet (52) when the other main surface (60t) of the base sheet (60) is suctioned through the conveyance surface (16s) of the conveyance device (16);
a fourth step of overlapping one main surface (62s) of a cover sheet (62) having the one main surface (62s) thereof applied with a second adhesive (82) with the one main surface (52s) of the fiber sheet (52) of the first laminate (50p) into the inside of which the absorbent material (2) is infiltrated, thereby forming a second laminate (50q) where the cover sheet (62) is laminated on the first laminate (50p); and
a fifth step of compressing the second laminate (50q) in a thickness direction by using compression means (19), and spreading the first adhesive (80) applied to the base sheet (60) and the second adhesive (82) applied to the cover sheet (62), in the inside of the fiber sheet (52) of the second laminate (50q).

2. The method of producing an absorbent body (50, 51) for an absorbent article according to claim 1, wherein the compression means (19) is a pair of rolls (19a, 19b), and compresses the second laminate (50q) in the thickness direction when the second laminate (50q) passes between the pair of rolls (19a, 19b).

3. The method of producing an absorbent body (50, 51) for an absorbent article according to claim 1 or 2, wherein the first and second adhesives (80, 82) contain a thermoplastic resin, and
the compression means (19) includes heating means for heating the second laminate (50q).

4. The method of producing an absorbent body (50, 51) for an absorbent article according to claim 1, wherein the first and second adhesives (80, 82) contain a thermoplastic resin,
the compression means (19) is a pair of rolls (19a, 19b), and compresses the second laminate (50q) in the thickness direction when the second laminate (50q) passes between the pair of rolls (19a, 19b), and
at least one of the pair of rolls (19a, 19b) is heated.

5. The method of producing an absorbent body (51) for an absorbent article according to any one of claims 1 to 4, the method further comprising:
a sixth step of supplying another base sheet (64) having one main surface (64s) thereof applied with a third adhesive (84), to another conveyance device (18), and conveying the other base sheet (64) while suctioning another main surface (64t) of the other base sheet (64) through a conveyance surface (16s) of the other conveyance device (18);
a seventh step of overlapping another main surface (54t) of another fiber sheet (54) having one main surface (54s) thereof nap-raised with the one main surface (64s) of the other base sheet (64) applied with the third adhesive (84), thereby forming a third laminate (50r) where the other fiber sheet (54) is laminated on the other base sheet (64);
an eighth step of scattering a granular other absorbent material (3) capable of absorbing and holding liquid, onto the one main surface (54s) of the other fiber sheet (54) of the third laminate (50r), and infiltrating the scattered other absorbent material (3) into an inside of the other fiber sheet (54) when the other main surface (64t) of the other base sheet (64) is suctioned through the conveyance surface (16s) of the other conveyance device (18);
a ninth step of applying a fourth adhesive (86) to the other main surface (60t) of the base sheet (60) of the second laminate (50q), and overlapping the other main surface (60t) of the base sheet (60) of the second laminate (50q) applied with the fourth adhesive (86) with the one main surface (54s) of the other fiber sheet (54) of the third laminate (50r) into the inside of which the other absorbent material (3) is infiltrated, thereby forming a fourth laminate (50s) where the second laminate (50q) is laminated on the third laminate (50r); and
a tenth step of compressing the fourth laminate (50s) in the thickness direction by using other compression means (19), and spreading the third adhesive (84) applied to the other base sheet (64) and the fourth adhesive (84) applied to the other main surface (60t) of the base sheet (60) of the second laminate (50q), in the inside of the other fiber sheet (54) of the fourth laminate (50s).

6. The method of producing an absorbent body (51) for an absorbent article according to claim 5, wherein when the fourth laminate (50s) is seen through in the thickness direction,
a plurality of first areas (56) where the absorbent material (2) is scattered so as to be continuous on the one main surface (52s) of the fiber sheet (52) are disposed at intervals in a conveyance direction of the fourth laminate (50s),
a plurality of second areas (58) where the other absorbent material (3) is scattered so as to be continuous on the one main surface (54s) of the other fiber sheet (54) are disposed at intervals in the conveyance direction,
each of the first areas (56) overlaps with only one of the second areas (58), respectively, and
the first areas (56) are narrower than the second areas (58) in the conveyance direction, or the second areas (58) are narrower than the first areas (56) in the conveyance direction.

7. A method of producing an absorbent article including the absorbent body (51) produced by the method of producing an absorbent body (51) for an absorbent article according to claim 6, comprising:
disposing the absorbent body (51) on the absorbent article in a manner that the fiber sheet (52) is on the skin side of the other fiber sheet (54) in a case that the first areas (56) are narrower than the second areas (58) in the conveyance direction when seen through in the thickness direction, or
disposing the absorbent body (51) on the absorbent article in a manner that the other fiber sheet (54) is on the skin side of the fiber sheet (52) in a case that the second areas (58) are narrower than the first areas (56) in the conveyance direction when seen through in the thickness direction.

## Patentansprüche

1. Verfahren zur Herstellung eines absorbierenden Körpers (50, 51) für einen absorbierenden Artikel, wobei das Verfahren umfasst:
einen ersten Schritt des Zuführens einer Basislage (60), deren eine Hauptoberfläche (60s) mit einem ersten Klebstoff (80) versehen ist, zu einer Fördervorrichtung (16), und des Förderns der Basislage (60), während eine andere Hauptoberfläche (60t) der Basislage (60) durch eine Förderoberfläche (16s) der Fördervorrichtung (16) gesaugt wird;
einen zweiten Schritt des Überlappens, entlang der Förderfläche (16s) der Fördervorrichtung (16), einer anderen Hauptoberfläche (52t) eines Faserblatts (52), dessen eine Hauptoberfläche (52s) mit der einen Hauptoberfläche (60s) des Basisblatts (60), die mit dem ersten Klebstoff (80) aufgebracht ist, angeraut ist, wodurch ein erstes Laminat (50p) gebildet wird, bei dem das Faserblatt (52) auf das Basisblatt (60) laminiert ist;
einen dritten Schritt des Streuens eines körnigen Absorptionsmaterials (2), das in der Lage ist, Flüssigkeit zu absorbieren und zu halten, auf die eine Hauptoberfläche (52s) der Faserlage (52) des ersten Laminats (50p), und des Infiltrierens des gestreuten Absorptionsmaterials (2) in das Innere der Faserlage (52), wenn die andere Hauptoberfläche (60t) der Basislage (60) durch die Förderoberfläche (16s) der Fördervorrichtung (16) gesaugt wird;
einen vierten Schritt des Überlappens einer Hauptoberfläche (62s) einer Decklage (62), deren eine Hauptoberfläche (62s) mit einem zweiten Klebstoff (82) versehen ist, mit der einen Hauptoberfläche (52s) der Faserlage (52) des ersten Laminats (50p), in dessen Inneres das absorbierende Material (2) infiltriert ist, wodurch ein zweites Laminat (50q) gebildet wird, bei dem die Decklage (62) auf das erste Laminat (50p) laminiert ist; und
einen fünften Schritt des Komprimierens des zweiten Laminats (50q) in einer Dickenrichtung unter Verwendung einer Kompressionseinrichtung (19) und des Verteilens des ersten Klebstoffs (80), der auf die Basislage (60) aufgebracht ist, und des zweiten Klebstoffs (82), der auf die Decklage (62) aufgebracht ist, im Inneren der Faserlage (52) des zweiten Laminats (50q).

2. Verfahren zur Herstellung eines absorbierenden Körpers (50, 51) für einen absorbierenden Artikel nach Anspruch 1, wobei das Kompressionsmittel (19) ein Paar von Rollen (19a, 19b) ist und das zweite Laminat (50q) in der Dickenrichtung komprimiert, wenn das zweite Laminat (50q) zwischen dem Paar von Rollen (19a, 19b) hindurchgeht.

3. Verfahren zur Herstellung eines absorbierenden Körpers (50, 51) für einen absorbierenden Artikel nach Anspruch 1 oder 2, wobei der erste und der zweite Klebstoff (80, 82) ein thermoplastisches Harz enthalten, und
die Kompressionseinrichtung (19) eine Heizeinrichtung zum Erwärmen des zweiten Laminats (50q) aufweist.

4. Verfahren zur Herstellung eines absorbierenden Körpers (50, 51) für einen absorbierenden Artikel nach Anspruch 1, wobei der erste und zweite Klebstoff (80, 82) ein thermoplastisches Harz enthalten,
das Kompressionsmittel (19) ein Paar von Walzen (19a, 19b) ist und das zweite Laminat (50q) in der Dickenrichtung komprimiert, wenn das zweite Laminat (50q) zwischen dem Paar von Walzen (19a, 19b) hindurchgeht, und
mindestens eine des Walzenpaars (19a, 19b) beheizt wird.

5. Verfahren zur Herstellung eines absorbierenden Körpers (51) für einen absorbierenden Artikel nach einem der Ansprüche 1 bis 4, wobei das Verfahren ferner umfasst:
einen sechsten Schritt des Zuführens einer anderen Basislage (64), deren eine Hauptoberfläche (62s) mit einem dritten Klebstoff (84) versehen ist, zu einer anderen Fördervorrichtung (18) und des Förderns der anderen Basislage (64), während eine andere Hauptoberfläche (64t) der anderen Basislage (64) durch eine Förderoberfläche (16s) der anderen Fördervorrichtung (18) gesaugt wird;
einen siebten Schritt des Überlappens einer anderen Hauptoberfläche (54t) eines anderen Faserbogens (54), dessen eine Hauptoberfläche (52s) mit der einen Hauptoberfläche (64s) des anderen Basisbogens (64), die mit dem dritten Klebstoff (84) aufgebracht ist, angeraut ist, wodurch ein drittes Laminat (50r) gebildet wird, bei dem der andere Faserbogen (54) auf den anderen Basisbogen (64) laminiert ist;
einen achten Schritt des Streuens eines körnigen anderen absorbierenden Materials (3), das in der Lage ist, Flüssigkeit zu absorbieren und zu halten, auf die eine Hauptoberfläche (54s) der anderen Faserlage (54) des dritten Laminats (50r), und des Infiltrierens des gestreuten anderen absorbierenden Materials (3) in das Innere der anderen Faserlage (54), wenn die andere Hauptoberfläche (64t) der anderen Basislage (64) durch die Förderoberfläche (16s) der anderen Fördervorrichtung (18) gesaugt wird;
einen neunten Schritt des Auftragens eines vierten Klebstoffs (86) auf die andere Hauptoberfläche (60t) der Basislage (60) des zweiten Laminats (50q), und
Überlappen der anderen Hauptoberfläche (60t) der Basislage (60) des zweiten Laminats (50q), auf die der vierte Klebstoff (86) aufgebracht ist, mit der einen Hauptoberfläche (54s) der anderen Faserlage (54) des dritten Laminats (50r), in deren Inneres das andere absorbierende Material (3) infiltriert ist, wodurch ein viertes Laminat (50s) gebildet wird, bei dem das zweite Laminat (50q) auf das dritte Laminat (50r) laminiert ist; und
einen zehnten Schritt des Komprimierens des vierten Laminats (50s) in der Dickenrichtung unter Verwendung anderer Kompressionsmittel (19) und des Verteilens des dritten Klebstoffs (84), der auf die andere Basislage (64) aufgebracht ist, und des vierten Klebstoffs (86), der auf die andere Hauptoberfläche (60t) der Basislage (60) des zweiten Laminats (50q) aufgebracht ist, im Inneren der anderen Faserlage (54) des vierten Laminats (50s).

6. Verfahren zum Herstellen eines absorbierenden Körpers (51) für einen absorbierenden Artikel nach Anspruch 5, wobei, wenn das vierte Laminat (50s) in der Dickenrichtung durchschaut wird,
eine Vielzahl von ersten Bereichen (56), in denen das absorbierende Material (2) so verstreut ist, dass es auf der einen Hauptoberfläche (52s) der Faserschicht (52) durchgehend ist, in Abständen in einer Transportrichtung des vierten Laminats (50s) angeordnet sind,
eine Vielzahl von zweiten Bereichen (58), in denen das andere absorbierende Material (3) so verstreut ist, dass es auf der einen Hauptoberfläche (54s) der anderen Faserlage (54) durchgehend ist, in Abständen in der Förderrichtung angeordnet sind,
jeder der ersten Bereiche (56) sich jeweils nur mit einem der zweiten Bereiche (58) überlappt und
die ersten Bereiche (56) schmaler sind als die zweiten Bereiche (58) in der Förderrichtung, oder die zweiten Bereiche (58) schmaler sind als die ersten Bereiche (56) in der Förderrichtung.

7. Verfahren zur Herstellung eines absorbierenden Artikels, der den absorbierenden Körper (51) enthält, der durch das Verfahren zur Herstellung eines absorbierenden Körpers (51) für einen absorbierenden Artikel gemäß Anspruch 6 hergestellt wurde, umfassend:
Anordnen des absorbierenden Körpers (51) auf dem absorbierenden Artikel in einer Weise, dass die Faserlage (52) auf der Hautseite der anderen Faserlage (54) in einem Fall ist, dass die ersten Bereiche (56) schmaler sind als die zweiten Bereiche (58) in der Förderrichtung, wenn man in der Dickenrichtung hindurchschaut, oder
Anordnen des absorbierenden Körpers (51) auf dem absorbierenden Artikel in einer Weise, dass sich die andere Faserlage (54) auf der Hautseite der Faserlage (52) befindet, wenn die zweiten Bereiche (58) schmaler sind als die ersten Bereiche (56) in der Förderrichtung, wenn man in der Dickenrichtung hindurchschaut.

## Revendications

1. Méthode de production d'un corps absorbant (50, 51) pour un article absorbant, la méthode comprenant :
une première étape consistant à fournir une feuille de base (60) dont une surface principale (60s) est appliquée avec un premier adhésif (80), à un dispositif de transport (16), et à transporter la feuille de base (60) tout en aspirant une autre surface principale (60t) de la feuille de base (60) à travers une surface de transport (16s) du dispositif de transport (16) ;
une deuxième étape de chevauchement, le long de la surface de transport (16s) du dispositif de transport (16), d'une autre surface principale (52t) d'une feuille de fibres (52) dont une surface principale (52s) est nappée avec la surface principale (60s) de la feuille de base (60) appliquée avec le premier adhésif (80), formant ainsi un premier stratifié (50p) dans lequel la feuille de fibres (52) est stratifiée sur la feuille de base (60) ;
une troisième étape consistant à disperser un matériau absorbant granulaire (2) capable d'absorber et de retenir un liquide sur l'une des surfaces principales (52s) de la feuille de fibres (52) du premier stratifié (50p), et à infiltrer le matériau absorbant dispersé (2) à l'intérieur de la feuille de fibres (52) lorsque l'autre surface principale (60t) de la feuille de base (60) est aspirée à travers la surface de transport (16s) du dispositif de transport (16) ;
une quatrième étape consistant à superposer une surface principale (62s) d'une feuille de couverture (62) dont la surface principale (62s) est appliquée avec un second adhésif (82) avec la surface principale (52s) de la feuille de fibres (52) du premier stratifié (50p) à l'intérieur duquel le matériau absorbant (2) est infiltré, formant ainsi un second stratifié (50q) dans lequel la feuille de couverture (62) est stratifiée sur le premier stratifié (50p) ; et
une cinquième étape consistant à comprimer le second stratifié (50q) dans le sens de l'épaisseur à l'aide de moyens de compression (19) et à étaler le premier adhésif (80) appliqué à la feuille de base (60) et le second adhésif (82) appliqué à la feuille de couverture (62) à l'intérieur de la feuille de fibres (52) du second stratifié (50q).

2. Procédé de fabrication d'un corps absorbant (50, 51) pour un article absorbant selon la revendication 1, dans lequel le moyen de compression (19) est une paire de rouleaux (19a, 19b), et comprime le second stratifié (50q) dans la direction de l'épaisseur lorsque le second stratifié (50q) passe entre la paire de rouleaux (19a, 19b).

3. Procédé de fabrication d'un corps absorbant (50, 51) pour un article absorbant selon la revendication 1 ou 2, dans lequel le premier et le second adhésifs (80, 82) contiennent une résine thermoplastique, et
le moyen de compression (19) comprend un moyen de chauffage pour chauffer le second stratifié (50q).

4. Procédé de fabrication d'un corps absorbant (50, 51) pour un article absorbant selon la revendication 1, dans lequel la première et la deuxième colle (80, 82) contiennent une résine thermoplastique,
le moyen de compression (19) est une paire de rouleaux (19a, 19b), et comprime le second stratifié (50q) dans la direction de l'épaisseur lorsque le second stratifié (50q) passe entre la paire de rouleaux (19a, 19b), et
au moins l'un des rouleaux (19a, 19b) est chauffé.

5. Procédé de fabrication d'un corps absorbant (51) pour un article absorbant selon l'une quelconque des revendications 1 à 4, le procédé comprenant en outre :
une sixième étape consistant à fournir une autre feuille de base (64) dont une surface principale (62s) est appliquée avec un troisième adhésif (84), à un autre dispositif de transport (18), et à transporter l'autre feuille de base (64) tout en aspirant une autre surface principale (64t) de l'autre feuille de base (64) à travers une surface de transport (16s) de l'autre dispositif de transport (18) ;
une septième étape consistant à superposer une autre surface principale (54t) d'une autre feuille de fibres (54) dont une surface principale (52s) est nappée avec une surface principale (64s) de l'autre feuille de base (64) appliquée avec le troisième adhésif (84), formant ainsi un troisième stratifié (50r) dans lequel l'autre feuille de fibres (54) est stratifiée sur l'autre feuille de base (64) ;
une huitième étape consistant à disperser un autre matériau absorbant granulaire (3) capable d'absorber et de retenir un liquide sur l'une des surfaces principales (54s) de l'autre feuille de fibres (54) du troisième stratifié (50r), et à infiltrer l'autre matériau absorbant dispersé (3) à l'intérieur de l'autre feuille de fibres (54) lorsque l'autre surface principale (64t) de l'autre feuille de base (64) est aspirée à travers la surface de transport (16s) de l'autre dispositif de transport (18) ;
une neuvième étape consistant à appliquer un quatrième adhésif (86) sur l'autre surface principale (60t) de la feuille de base (60) du second stratifié (50q), et le chevauchement de l'autre surface principale (60t) de la feuille de base (60) du deuxième stratifié (50q) appliquée avec le quatrième adhésif (86) avec l'une des surfaces principales (54s) de l'autre feuille de fibres (54) du troisième stratifié (50r) à l'intérieur duquel l'autre matériau absorbant (3) est infiltré, formant ainsi un quatrième stratifié (50s) dans lequel le deuxième stratifié (50q) est stratifié sur le troisième stratifié (50r) ; et
une dixième étape consistant à comprimer le quatrième stratifié (50s) dans le sens de l'épaisseur à l'aide d'autres moyens de compression (19) et à étaler le troisième adhésif (84) appliqué à l'autre feuille de base (64) et le quatrième adhésif (86) appliqué à l'autre surface principale (60t) de la feuille de base (60) du deuxième stratifié (50q), à l'intérieur de l'autre feuille de fibres (54) du quatrième stratifié (50s).

6. Procédé de fabrication d'un corps absorbant (51) pour un article absorbant selon la revendication 5, dans lequel, lorsque le quatrième stratifié (50s) est vu à travers dans la direction de l'épaisseur,
une pluralité de premières zones (56) où le matériau absorbant (2) est dispersé de manière à être continu sur la surface principale (52s) de la feuille de fibres (52) sont disposées à intervalles dans une direction de transport du quatrième stratifié (50s),
une pluralité de secondes zones (58) où l'autre matériau absorbant (3) est dispersé de manière à être continu sur l'une des surfaces principales (54s) de l'autre feuille de fibres (54) sont disposées à intervalles dans la direction d'acheminement,
chacune des premières zones (56) ne recouvre qu'une seule des deuxièmes zones (58), respectivement, et
les premières zones (56) sont plus étroites que les secondes zones (58) dans la direction de transport, ou les secondes zones (58) sont plus étroites que les premières zones (56) dans la direction de transport.

7. Procédé de production d'un article absorbant comprenant le corps absorbant (51) produit par le procédé de production d'un corps absorbant (51) pour un article absorbant selon la revendication 6, comprenant :
disposer le corps absorbant (51) sur l'article absorbant de manière à ce que la feuille de fibres (52) se trouve du côté de la peau de l'autre feuille de fibres (54) dans un cas où les premières zones (56) sont plus étroites que les secondes zones (58) dans la direction de transport lorsqu'elles sont vues à travers dans la direction de l'épaisseur, ou
en disposant le corps absorbant (51) sur l'article absorbant de telle sorte que l'autre feuille de fibres (54) se trouve du côté de la peau de la feuille de fibres (52) dans le cas où les deuxièmes zones (58) sont plus étroites que les premières zones (56) dans la direction de transport lorsqu'elles sont vues à travers dans la direction de l'épaisseur.
